# FASCICULE DE BREVET EUROPEEN

(11) **EP 3 758 709 B1**
(45) Date de publication et mention de la délivrance du brevet: **09.07.2025**
(21) Numéro de dépôt: 19711977.9
(22) Date de dépôt: 20.02.2019
(51) Int. Cl.: A61K 31/575, A61P 21/06

(54) **PHYTOECDYSONES POUR LEUR UTILISATION DANS LA PRÉVENTION DE LA PERTE DE FORCE MUSCULAIRE LORS D'UNE IMMOBILISATION**
PHYTOECDYSONE ZUR VERWENDUNG BEI DER PRÄVENTION VON MUSKELKRAFTVERLUST WÄHREND DER IMMOBILISIERUNG
PHYTOECDYSONES FOR USE IN THE PREVENTION OF MUSCLE STRENGTH LOSS DURING IMMOBILISATION

(30) Priorité: 28.02.2018 FR 1851778
(43) Date de publication de la demande: 06.01.2021
(73) Titulaire: Biophytis, 75001 Paris (FR); SORBONNE UNIVERSITE, 75006 Paris (FR)
(72) Inventeur: DILDA, Pierre, 75016 PARIS (FR); LATIL, Mathilde, 75019 PARIS (FR); LAFONT, René, 75016 PARIS (FR); VEILLET, Stanislas, 91600 SAVIGNY SUR ORGE (FR)
(74) Mandataire: Ipside
(86) Numéro de dépôt international: PCT/FR2019/050392
(87) Numéro de publication internationale: WO 2019/166717

(56) Documents cités:
- WO-A1-2015/177469
- WO-A1-2016/166480
- FR-A1- 2 983 733

## Description

### DOMAINE TECHNIQUE DE L'INVENTION

La présente invention concerne l'utilisation de phytoecdysones et de leurs dérivés pour utilisation dans la prévention de la perte de force musculaire lors d'une immobilisation.

### ÉTAT DE LA TECHNIQUE

Le risque de complications suite à l'immobilisation d'un membre ou à la tenue d'une position allongée sur le long terme (décubitus) est important et peut se manifester par des troubles musculaires, mais aussi broncho-pulmonaires, cardio-vasculaires ou ostéoarticulaires (Bodine 2013, Hermans & Van den Berghe 2015 ; De Jonghe et al. 2007 ; Wentworth et al. 2017 ; lordens et al. 2017).

L'immobilisation peut être progressive ou brutale et peut concerner un membre ou bien s'étendre à tout le corps dans les cas les plus extrêmes. Les circonstances aboutissant à une immobilisation sont multiples, par exemple :
- la fracture ou blessure d'un membre conduisant à la mise en place d'un dispositif d'immobilisation externe (contentions non plâtrées de type minerve, écharpe, bandage de type Dujarrier, attelles métalliques ou de Zimmer, syndactylie ou contentions plâtrées avec immobilisation complète ou incomplète d'un membre),
- une rupture ligamentaire partielle ou totale nécessitant ou non une intervention chirurgicale conduisant à la mise en place d'un dispositif d'immobilisation externe,
- la pose d'une prothèse de hanche,
- la pose d'une prothèse de genou,
- les montages orthopédiques ne permettant pas l'appui immédiat,
- la fracture du bassin en période douloureuse,
- la facture du col du fémur opérée ou non opérée, stable ou instable,
- l'atteinte nerveuse périphérique partielle due à un traumatisme,
- l'atteinte partielle de la moelle épinière due à un traumatisme, et
- les patients placés de façon prolongée en position de décubitus.

Dans tous les cas, l'immobilisation va conduire à des altérations du tissu musculaire. On observe une diminution de la masse musculaire, une fonte musculaire ou amyotrophie, ainsi qu'une diminution de la force et de la puissance musculaire ce qui conduit généralement à une période d'incapacité et dans certains cas à un allongement de la prise en charge occasionnant une augmentation des coûts hospitaliers.

L'atrophie et la perte de force du muscle squelettique suite à l'immobilisation ont des conséquences fonctionnelles notamment sur la posture et l'équilibre, ce qui augmente le risque de chute, notamment chez les personnes âgées (Onambele et al., 2006).

En France, on estime entre 50 000 et 80 000 par an le nombre de fractures de l'extrémité supérieure du fémur chez les personnes âgées. La très grande majorité de ces fractures du fémur sont consécutives à des chutes (INSERM 2015). Les fractures du col de fémur conduisent à une baisse transitoire de la mobilité, pouvant entrainer la survenue de complications. La fracture de l'extrémité du fémur est une des principales causes de mortalité chez les plus de 65 ans (Gillespie et al. 2012). Dans l'année qui suit l'accident, la mortalité est de 10 à 20% plus élevée que celle de sujets de même âge et de même sexe. Toutes causes confondues, de 20 à 30% des patients de plus de 55 ans décèdent dans l'année qui suit une fracture de l'extrémité supérieure du fémur (Klop et al., 2014 et Lund et al., 2014).

Chez le sujet âgé une atrophie musculaire suite à une immobilisation a de très sérieuses conséquences qui peuvent être aggravées par une atrophie musculaire en liaison avec le vieillissement (sarcopénie). Les mécanismes mis en œuvre dans l'atrophie musculaire liée au vieillissement et dans l'atrophie musculaire liée à une immobilisation sont cependant différents (revues dans Lynch et al., 2007 ; Romanick & Brown-Borg, 2013).

Lors d'une immobilisation, on peut observer que ce sont les fibres de type I (oxydatives) qui sont particulièrement affectées par l'atrophie (Bigard et al., 1998; Ohira et al., 2006). En revanche, la sarcopénie se manifeste par une atrophie particulière concernant les fibres de type II (glycolytiques), associée au développement du tissu conjonctif (fibrose) ainsi qu'à une infiltration par des cellules adipeuses (ex. Nilwik et al., 2013). La sarcopénie est caractérisée par une diminution du diamètre des fibres et de leur nombre (Lexell, 1993). En revanche, dans un contexte d'atrophie liée à une immobilisation, la taille des fibres diminue mais le nombre de fibres musculaire reste constant (Narici, 2010). De plus, l'atrophie met en jeu des processus d'autophagie dont la régulation diffère selon le type de fibres (Yamada et al., 2012).

Les mécanismes moléculaires qui sous-tendent l'atrophie musculaire induite sont également différents. Certains gènes connus pour induire une atrophie musculaire, tels que MuRF1 et Atrogine, peuvent être activés par des voies de signalisation bien connues telle que NF- 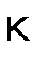 B. Cette voie de signalisation est activée dans des conditions d'atrophie liée à la cachexie ou à l'immobilisation (Hunter et al., 2002) mais pas dans un contexte de sarcopénie (Bar-Shai et al., 2005 ; Phillips, 2005 ; Sakuma, 2012).

La myostatine, un régulateur négatif de la croissance musculaire, augmente lors de l'atrophie liée à la cachexie et lors d'une immobilisation mais il a été démontré que cela n'était pas le cas chez des animaux âgés, chez lesquels le niveau de myostatine restait relativement inchangé (Siriett et al., 2006 ; Lebrasseur et al., 2009).

Dans ces conditions, le fait qu'une substance soit efficace pour traiter la sarcopénie ne permet pas de présager qu'elle sera efficace pour prévenir les troubles musculaires liés à l'immobilisation.

L'immobilisation a également un impact sur la récupération des performances physiques maximales, notamment chez les athlètes (Milsom et al. 2014).

L'immobilisation plâtrée d'un membre blessé entraine des modifications structurales des muscles concernés par l'immobilisation. Pour exemple, deux mois d'immobilisation de la cheville conduisent à une diminution du volume du triceps sural et du quadriceps respectivement de 21.9% et 24.1%. Deux mois après la levée de l'immobilisation, les deux muscles sont toujours 9.5% et 5.2% moins volumineux qu'avant immobilisation (Grosset & Onambele-Pearson, 2008). En termes de surface de fibres musculaires (en anglais CSA pour *cross-sectional area*), cinq semaines d'immobilisation réduisent la CSA de 10 à 20% suivant le type de fibres et les muscles concernés (Suetta et al. 2004 ; Berg et al. 2007). La CSA de muscles supportant le poids corporel diminue approximativement de 2-3% par semaine durant les premiers mois d'immobilisation (Berg et al. 2007).

La force musculaire est également fortement diminuée suite à une immobilisation. De manière générale, une immobilisation de la jambe pendant deux semaines produit une perte d'un tiers de la force musculaire chez les jeunes, tandis que les sujets plus âgés perdent environ un quart de leur force. Chez ces derniers, la perte de puissance musculaire peut s'avérer irréversible. Ceci peut être à l'origine d'une perte de confiance et d'une fragilité qui aboutissent invariablement à la dépendance. De plus, l'apparition d'un syndrome d'immobilisation peut être consécutive à un alitement ou simplement à une forte réduction d'activité.

Des patients alités pendant plus d'une semaine ont une perte de force musculaire de leurs muscles antigravitationnels des mollets et du dos (Hermans et Van den Berghe, 2015). Chez l'homme sain, durant la première semaine d'alitement, des pertes de force comprises entre 1% et 6% par jour ont été observées (Appell, 1990). Une période de 1 à 2 semaines d'inactivité des membres inférieurs induit une réduction de 15% de la force des extenseurs du genou chez des sujets d'une vingtaine d'années.

De plus, une étude a démontré que la pose d'un plâtre pendant 5 jours induisait une perte de force du quadriceps de 9% tandis que cette perte atteint 23% après 14 jours d'immobilisation. Un alitement de 3 mois à des effets très marqués sur la force développée avec une diminution de 31% à 60% en fonction du muscle considéré (Alkner & Tesch, 2004).

La mise en évidence du rôle délétère de l'immobilisation musculaire a amené à tester la faisabilité et l'efficacité de plusieurs méthodes préventives. Ainsi, dans certains cas, afin de pallier les dommages musculaires provoqués par l'immobilisation, des programmes de remobilisation active ou passive précoces des patients sont mis en place. Ces programmes présentent l'inconvénient d'être difficiles à instaurer à grande échelle. De plus, l'utilisation de ces méthodes est délicate, voire impossible chez certains patients chirurgicaux chez qui la mobilisation s'avère douloureuse.

Des études d'électrostimulation musculaire ont également été menées, mais cette approche ne permet pas d'avoir un effet bénéfique systémique de la stimulation, elle peut s'avérer inefficace chez des patients présentant une inexcitabilité de la membrane musculaire et le choix du territoire musculaire à stimuler est délicat dans les cas d'immobilisation étendue.

Outre les modifications tissulaires et mécaniques du muscle lors d'une immobilisation, il apparait également des changements métaboliques et moléculaires majeurs.

Ainsi, la synthèse protéique est diminuée, tandis que l'immobilisation provoque une augmentation du stress oxydatif, de l'inflammation, de l'apoptose ainsi que l'activation de voies protéolytiques qui conduisent à la dégradation des protéines musculaires.

Les phytoecdysones représentent une importante famille de stérols polyhydroxylés. Ces molécules sont produites par diverses espèces de plantes et participent à la défense de ces plantes contre les ravageurs. La phytoecdysone majoritaire du règne végétal est la 20-hydroxyecdysone.

Des études ont mis en avant les propriétés antidiabétiques et anabolisantes de certaines phytoecdysones. Des effets stimulants sur les synthèses protéiques dans les muscles sont observés chez le rat *in vivo* (Syrov, 2000; Tôth et al., 2008; Lawrence, 2012) et sur des myotubes murins C2C12 in vitro (Gorelick-Feldman et al., 2008).

Des dérivés hémi-synthétiques de la 20-hydroxyecdysone ont été proposés dans la publication de la demande de brevet français de la société demanderesse, publiée sous le numéro FR3021318A1, et dans la demande de brevet internationale WO 2015/177469. Leur utilisation en tant que médicament pour le traitement et/ou la prévention de la sarcopénie et en particulier de l'obésité sarcopénique, de ses complications et/ou pathologies associées telles que la perte de force, de masse musculaire, de performances et capacité physiques et de mobilité chez le mammifère y est également mentionnée.

En outre, la demande de brevet internationale WO2016/166480 divulgue l'utilisation de composés dérivés de 20-hydroxyecdysone pour le traitement ou la prévention de l'atrophie musculaire chez les mammifères, cette atrophie musculaire pouvant être liée à une immobilisation.

### OBJET DE L'INVENTION

La présente invention a pour objectif de limiter le plus possible la perte de force musculaire lors d'une immobilisation notamment consécutive par exemple à une fracture, un alitement ou simplement à une forte réduction d'activité.

Les inventeurs ont découvert que les phytoecdysones et leurs dérivés protègent contre la perte de force musculaire liée à une immobilisation. La force musculaire est définie par la force maximale isométrique absolue et spécifique du muscle squelettique.

De manière inattendue, les phytoecdysones et leurs dérivés diminuent de manière significative la perte de force musculaire liée à une immobilisation sans pour autant que cette propriété soit reliée à un effet anabolisant sur le muscle squelettique.

Dans la suite de la description, on entend par phytoecdysones et leurs dérivés des extraits de végétaux riches en 20-hydroxyecdysone, et des compositions comportant à titre d'agent actif la 20-hydroxyecdysone.

Lesdits extraits de végétaux riches en 20-hydroxyecdysone sont par exemple des extraits de Stemmacantha carthamoides ou de Cyanotis vaga.

Les extraits obtenus sont de préférence purifiés au grade pharmaceutique.

L'invention concerne une composition comportant au moins :
- la 20-hydroxyecdysone ; ou,
- au moins un composé de formule générale (I) : 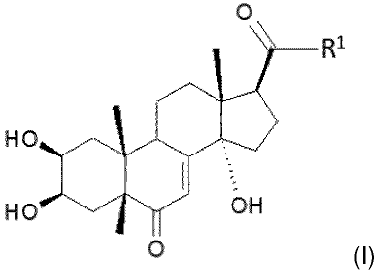 dans lequel :
   **R¹** est choisi parmi : un groupement (C₁-C₆)**W**(C₁-C₆) ; un groupement (C₁-C₆)**W**(C₁-C₆)**W**(C₁-C₆); un groupement (C₁-C₆)**W**(C₁-C₆)CO₂(C₁-C₆); un groupement (C₁-C₆)**A**, **A** représentant un hétérocycle éventuellement substitué par un groupement choisi parmi OH, OMe, (C₁-C₆), N(C₁-C₆), CO₂(C₁-C₆) ; un groupement CH₂Br,
      **W** étant un hétéroatome choisi parmi N, O et S, de préférence O et encore plus préférentiellement S ; ou,
- au moins un composé de formule (II) : 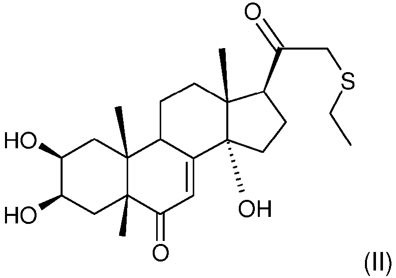 pour son utilisation chez le mammifère pour la prévention de la perte de force musculaire lors de l'immobilisation.

Un exemple d'une telle composition est l'extrait purifié au grade pharmaceutique BIO101 ayant été développé par le demandeur. BIO101 est un extrait de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone.

Dans des modes de réalisation la composition comporte un composé choisi parmi les composés suivants :
**n° 1** : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one,
**n° 2** : (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
**n° 3** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
**n° 4 :** (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
**n° 5 :** (2S,3R,5R,10R,13R,14S,17S)-17-[2-(3-diméthylaminopropyl (méthyl)amino)acétyl]-2,3,14-trihydroxy-10,13-diméthyl-2,3,4,5,9,11,12,15,16,17-décahydro-1H-cyclopenta[a]phénanthrèn-6-one;
**n° 6 :** 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
**n° 7** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
**n° 8 :** (2S,3R,5R,10R,13R,14S,17S)-2,3,14-trihydroxy-17-[2-(2-hydroxyéthyl sulfanyl)acétyl]-10,13-diméthyl-2,3,4,5,9,11,12,15,16,17-décahydro-1H cyclopenta[a]phénanthrèn-6-one.

Dans des modes de réalisation la composition est incorporée à une formulation pharmaceutique acceptable pouvant être administrée par voie orale.

Dans des modes de réalisation, les phytoecdysones sont administrées à une dose comprise entre 50 et 1000 milligrammes par jour chez l'humain.

Dans des modes de réalisation, le composé de formule (II) est administré à une dose comprise entre 50 et 1000 milligrammes par jour chez l'humain.

Dans des modes de réalisation, la composition est administrée lors de l'immobilisation. Préférentiellement, la composition est administrée dès le premier jour de l'immobilisation.

Dans des modes de réalisation, la composition est administrée jusqu'à la levée de l'immobilisation.

Dans des modes de réalisation, la composition est administrée en outre au cours d'une durée prédéterminée après la levée de l'immobilisation.

Dans un exemple de réalisation, la durée prédéterminée de traitement après la levée de l'immobilisation correspond au temps nécessaire à la récupération d'un seuil de force correspondant par exemple à 80% ou 100% de la force initiale estimée du sujet.

Dans un exemple de réalisation, la durée prédéterminée de traitement correspond à une période d'au moins 28 jours.

Dans un exemple de réalisation, la durée prédéterminée de traitement correspond à une période de trois à six mois.

Préférentiellement, le traitement après la levée de l'immobilisation est complémenté par un programme d'exercices physiques.

### PRÉSENTATION DES FIGURES

D'autres avantages, buts et caractéristiques particuliers de la présente invention ressortiront de la description non limitative qui suit d'au moins un mode de réalisation particulier de l'objet de la présente invention, en regard des dessins annexés, dans lesquels :

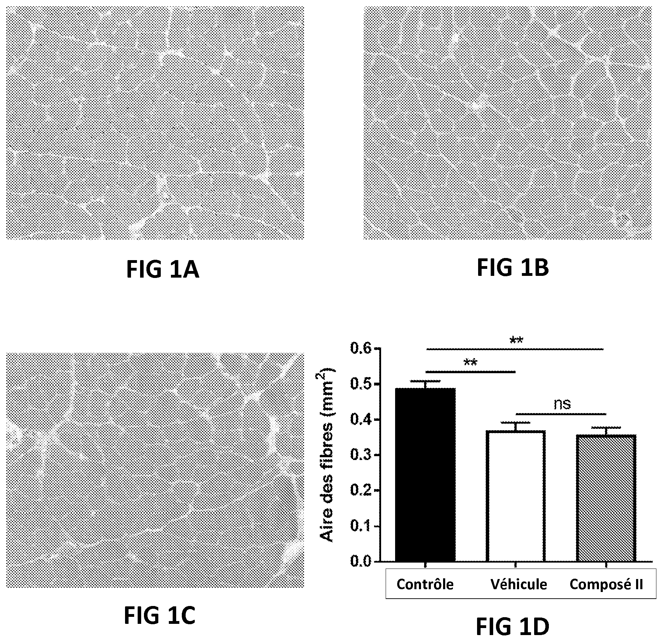

- La figure 1A est une image représentative de coupes histologiques colorées à l'hématoxyline Eosine de muscle tibial antérieur (TA) de souris de fond génétique C57BL/6J non immobilisée,
- La figure 1B est une image représentative de coupes histologiques colorées à l'hématoxyline Eosine de muscle tibial antérieur de souris de fond génétique C57BL/6J immobilisée et traitée avec le véhicule pendant 14 jours,
- La figure 1C est une image représentative de coupes histologiques colorées à l'hématoxyline Eosine de muscle tibial antérieur de souris de fond génétique C57BL/6J immobilisée et traitée avec le composé de formule (II) pendant 14 jours.
- La figure 1D est un diagramme représentatif de l'aire des fibres musculaires du muscle tibial antérieur de souris de fond génétique C57BL/6J non immobilisées (contrôle), immobilisées et traitées avec le véhicule pendant 14 jours ou immobilisées et traitées avec un composé de formule (II) pendant 14 jours.

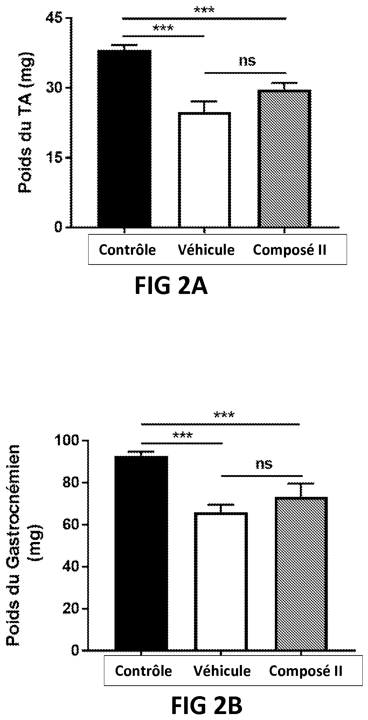

- La figure 2A est un diagramme représentatif du poids du muscle tibial antérieur des groupes de souris de fond génétique C57BL/6J non immobilisées (contrôle), immobilisées et traitées avec le véhicule pendant 14 jours ou immobilisées et traitées avec le composé de formule (II) pendant 14 jours.
- La figure 2B est un diagramme représentatif du poids du muscle gastrocnémien des groupes de souris de fond génétique C57BL/6J non immobilisées (contrôle), immobilisées et traitées avec le véhicule pendant 14 jours ou immobilisées et traitées avec le composé de formule (II) pendant 14 jours.

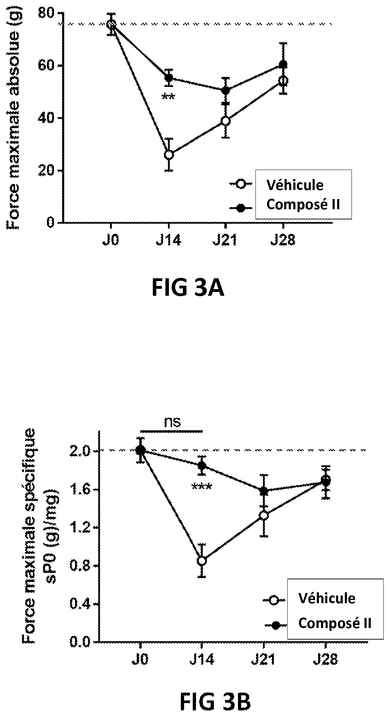

- La figure 3A représente la force isométrique maximale absolue du muscle tibial antérieur de souris de fond génétique C57BL/6J à différents temps post immobilisation : non immobilisées (J0), après 14 jours d'immobilisation (J14), après 14 jours d'immobilisation et 1 semaine de remobilisation (J21) ou après 14 jours d'immobilisation et 2 semaines de remobilisation (J28), traitées par le véhicule ou par le composé de formule (II).
- La figure 3B représente la force isométrique maximale spécifique du muscle tibial antérieur de souris de fond génétique C57BL/6J à différents temps post immobilisation : non immobilisées (J0), après 14 jours d'immobilisation (J14), après 14 jours d'immobilisation et 1 semaine de remobilisation (J21) ou après 14 jours d'immobilisation et 2 semaines de remobilisation (J28), traitées par le véhicule ou par le composé de formule (II).

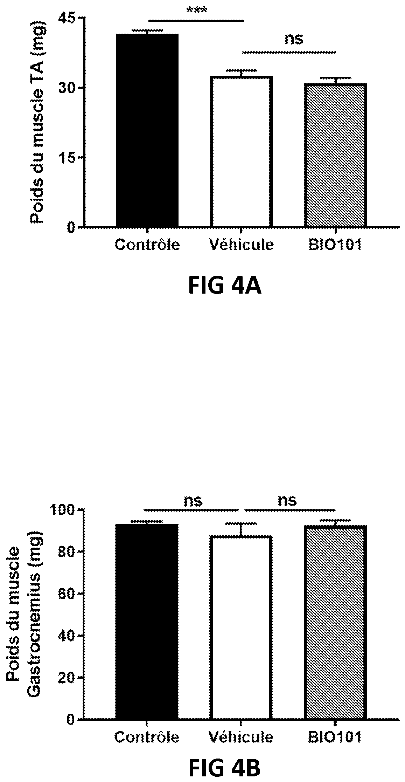

- La figure 4A est un diagramme représentatif du poids du muscle tibial antérieur des groupes de souris de fond génétique C57BL/6J non immobilisées (contrôle, mesuré à J0 sur des animaux non immobilisés), immobilisées et traitées avec le véhicule pendant 14 jours ou immobilisées et traitées avec le composé BIO101 pendant 14 jours.
- La figure 4B est un diagramme représentatif du poids du muscle gastrocnémien des groupes de souris de fond génétique C57BL/6J non immobilisées (contrôle), immobilisées et traitées avec le véhicule pendant 14 jours ou immobilisées et traitées avec le composé BIO101 pendant 14 jours.

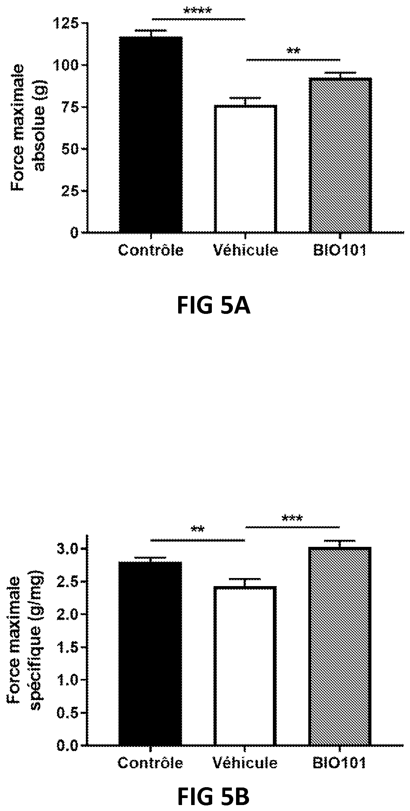

- La figure 5A est un diagramme représentatif de la force isométrique maximale absolue du muscle tibial antérieur de souris de fond génétique C57BL/6J non immobilisées (contrôle, mesuré à J0 sur des animaux non immobilisés), immobilisées et traitées avec le véhicule pendant 7 jours ou immobilisées et traitées avec le composé BIO101 pendant 7 jours.
- La figure 5B est un diagramme représentatif de la force isométrique maximale spécifique du muscle tibial antérieur de souris de fond génétique C57BL/6J non immobilisées (contrôle, mesuré à J0 sur des animaux non immobilisés), immobilisées et traitées avec le véhicule pendant 7 jours ou immobilisées et traitées avec le composé BIO101 pendant 7 jours.

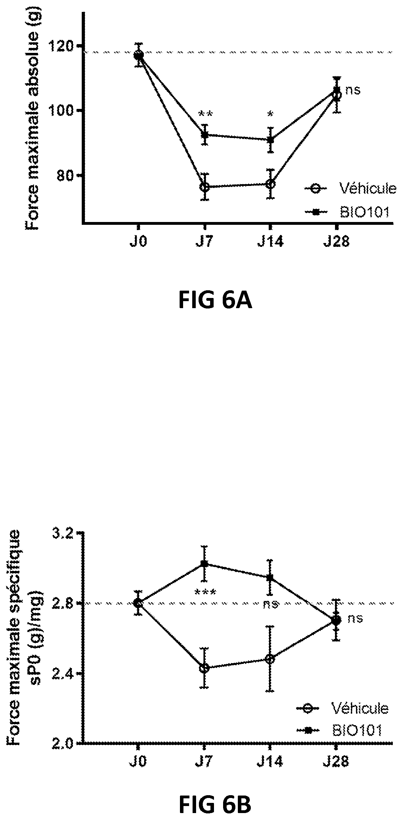

- La figure 6A représente la force isométrique maximale absolue du muscle tibial antérieur de souris de fond génétique C57BL/6J à différents temps post immobilisation : non immobilisées (J0), après 7 jours d'immobilisation (J7), après 14 jours d'immobilisation (J14), et après 14 jours d'immobilisation puis 2 semaines de remobilisation (J28), traitées par le véhicule ou par le composé BIO101.
- La figure 6B représente la force isométrique maximale spécifique du muscle tibial antérieur de souris de fond génétique C57BL/6J à différents temps post immobilisation : non immobilisées (J0), après 7 jours d'immobilisation (J7), après 14 jours d'immobilisation (J14), après 14 jours d'immobilisation puis 2 semaines de remobilisation (J28), traitées par le véhicule ou par le composé BIO101.

### DESCRIPTION DÉTAILLÉE DE L'INVENTION

### Procédé de synthèse du composé de formule (II)

Le composé de formule (II) auquel il est fait référence dans le reste de la description est le suivant:

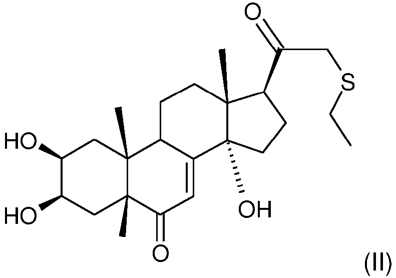

Le composé de formule (II) est obtenu par hémi-synthèse à partir de 20-hydroxyecdysone puis purification au grade pharmaceutique.

Le procédé de préparation du composé de formule (II) par hémi-synthèse comporte notamment:
- une étape de coupure oxydante de la chaîne latérale de la 20-hydroxyecdysone entre les carbones C20 et C22 pour obtenir la poststérone,
- une étape d"introduction d'un atome de brome en position C21, et
- une étape de réaction du dérivé bromé avec de l'éthane-thiol.

### Activité biologique du composé de formule (II)

Un modèle d'immobilisation d'une patte postérieure de souris de fond génétique C57BL/6J a été mis en œuvre à l'aide d'un tube (Lang et al., 2012).

Des souris C57BL/6J femelles âgées de 13 semaines ont été utilisées. Dix souris ont été sacrifiées à J0, ces souris n'ont pas été immobilisées afin de servir de contrôle.

On entend par J0, J14, J21, J28 le temps écoulé à compter du démarrage de l'expérimentation, exprimé en jours. Ainsi, J0 désigne le début de l'expérimentation (avant traitement et avant immobilisation), J14 désigne le 14ème jour à compter du début de l'expérimentation, etc..

Deux groupes des souris ont été constitués, un groupe de test et un groupe témoin. Chaque groupe est exposé, par voie orale, de manière chronique soit au véhicule (groupe témoin) soit au composé de formule (II) à la dose de 50 mg/kg par jour (groupe test). Le traitement par voie orale sur 28 jours consiste au gavage pendant cinq jours par semaine et dans l'eau de boisson deux jours par semaine.

Les animaux de tous les groupes ont été testés pour leur capacité fonctionnelle in situ grâce à des mesures de la force maximale isométrique absolue et spécifique du muscle tibial antérieur (TA) (Figures 3A et 3B) après 14 jours d'immobilisation (n=13 pour le véhicule, n=10 pour le composé de formule (II) ), après 14 jours d'immobilisation et 1 semaine de remobilisation (n=7 pour le véhicule, n=8 pour le composé de formule (II)) et après 14 jours d'immobilisation et deux semaines de remobilisation (n=6 pour le véhicule, n=8 pour le composé de formule (II)).

### Histologie et atrophie des muscles (Fiqure 1)

Une étude histologique du muscle tibial antérieur est réalisée sur des coupes colorées à l'hématoxyline Eosine (HE). L'aire des fibres musculaires est évaluée sur des muscles de souris contrôle, ou traitée avec le véhicule ou avec le composé de formule (II). Le muscle présente dans tous les cas une histologie de tissu musculaire sain (figure 1 A à C); en revanche, comme on pouvait s'y attendre, après 14 jours d'immobilisation, l'aire moyenne des fibres est fortement diminuée chez les animaux ayant reçu le véhicule par rapport aux animaux contrôle (-24,4 %, p=0,006) qui n'ont pas été immobilisées. L'aire des fibres musculaires du groupe traité avec le composé de formule (II)) est également diminuée par rapport au groupe contrôle (-26,8%, p=0,002).

On n'observe donc pas de différence significative entre les groupes d'animaux traités avec le véhicule et le groupe traité avec composé de formule (II) (p=0,73). Après 14 jours d'immobilisation, le composé de formule (II) n'exerce donc pas d'effet protecteur contre la perte de volume musculaire.

### Poids des muscles tibial antérieur (TA) et gastrocnémien (Figure 2)

Le poids des muscles TA (Figure 2A) et gastrocnémien (Figure 2B) ont été évalués chez des souris non immobilisées (contrôle), et après 14 jours d'immobilisation chez des souris traitées soient par le véhicule soit par le composé de formule (II), pendant les 14 jours d'immobilisation.

De manière attendue, on observe que l'immobilisation provoque une diminution de la masse musculaire du TA et du gastrocnémien chez des souris ayant reçu le véhicule par rapport au groupe contrôle (-34,9%, p<0,001 et -29%, p<0,001 respectivement).

On observe que le poids des muscles TA et gastrocnémien ne varie pas significativement dans le groupe des souris traitées avec le composé de formule (II) par rapport au véhicule. En cohérence avec les résultats obtenus sur le diamètre des fibres musculaires (Figure 1), le composé de formule (II) n'exerce donc pas d'effet protecteur contre la perte de masse musculaire suite à une immobilisation.

### Force maximale isométrique absolue et spécifique du muscle tibial antérieur (étude fonctionnelle in situ (Figure 3)

Une évaluation de la contractilité in situ du muscle TA est réalisée à différents temps du protocole : sur des souris contrôles non immobilisées (J0), sur des souris soumises à une immobilisation de la patte postérieure pendant 14 jours (J14), immobilisées 14 jours puis remobilisées 1 semaine (J21) et immobilisées 14 jours puis remobilisées 2 semaines (J28).

Le jour du sacrifice, la souris est anesthésiée avec une injection intrapéritonéale de pentobarbital (55 mg/kg, 0,1 mL/10 g de poids corporel) avant la mesure de force in situ du muscle tibial antérieur (TA). La peau du dessus de la patte est incisée, ce qui laisse apparaître le tendon qui est sectionné à son extrémité distale. Le tendon distal du TA est attaché au levier du servomoteur (305B Dual-Mode Lever, Aurora Scientific). La peau sur la face latérale de la cuisse est incisée, ce qui laisse apparaître le nerf sciatique, entre deux groupes musculaires. Le nerf sciatique est stimulé avec une électrode bipolaire (pulse supra-maximal d'onde carrée de 10V, 0,1 ms). La force est mesurée pendant des contractions en réponse à la stimulation électrique (fréquence de 75-150 Hz, durée de 500 ms). La température de la souris est maintenue à 37°C à l'aide de lampe radiante. La force maximale isométrique absolue est mesurée (Figure 3A) et la force maximale isométrique spécifique (Figure 3B) est calculée en rapportant la force isométrique absolue au poids du muscle tibial antérieur.

De manière attendue, on constate que les animaux traités avec le véhicule ont une force de contraction maximale isométrique absolue significativement moins importante que celle des animaux contrôle non immobilisés (-65,6%, p<0,001) (Figure 3A). Les animaux traités avec le composé de formule (II) présentent une perte de force absolue moins importante (-26,9%, p=0,015) par rapport au contrôle, que les animaux traités avec le véhicule.

De manière surprenante, on observe que le traitement par le composé de formule (II) permet aux animaux immobilisés pendant 14 jours de conserver une force isométrique absolue significativement plus importante que les animaux traités par le véhicule et améliore leur performance (+112,1%, p=0,0041). Ce malgré l'absence d'effet du composé de formule (II) sur la parte de masse et de volume musculaire constatée précédemment.

On observe que les animaux traités avec le véhicule ont une force de contraction maximale isométrique spécifique (sP0 ; Figure 3B) significativement moins importante que celle des animaux contrôle non immobilisés (-57,8%, p<0,001). De manière remarquable, la force spécifique des animaux traités avec le composé de formule (II) n'est pas significativement affectée par l'immobilisation : -8% (p=0,32) par rapport aux animaux du groupe contrôle, non immobilisés. Le traitement par le composé de formule (II) permet aux animaux immobilisés pendant 14 jours de conserver une fonction musculaire normale en doublant la force isométrique spécifique (+117,6%, p<0,001) par rapport aux animaux du groupe immobilisés, traités avec le véhicule.

### Activité biologique du composé BIO101

Une seconde étude a été réalisée en utilisant le même procédé d'immobilisation des deux pattes postérieures, sur des souris du même âge (13 semaines) et du même fond génétique (C57BL/6J) que décrit précédemment, mais en rajoutant un point d'analyse après 7 jours d'immobilisation. Les points d'analyse sont donc J0, J7, J14 et J28.

Dix souris ont été sacrifiées à J0, ces souris n'ont pas été immobilisées afin de servir de contrôles (groupe contrôle dans les figures).

On entend par J7, J14, J28 le temps écoulé à compter du démarrage de l'expérimentation, exprimé en jours. Ainsi, J7 désigne le 7ème jour à compter du début de l'expérimentation, etc.

Deux groupes de souris ont été constitués, un groupe de test et un groupe témoin. Chaque groupe est exposé, par voie orale, de manière chronique soit au véhicule (groupe témoin) soit au composé BIO101 à la dose de 50 mg/kg par jour (groupe test). On entend par composé BIO101 un extrait de plante, ladite plante étant choisie parmi les végétaux contenant au moins 0,5% de 20-hydroxyecdysone en poids sec dudit végétal, ledit extrait comportant à titre d'agent actif la 20-hydroxyecdysone en quantité d'au moins 95%, et de préférence au moins 97% en poids par rapport au poids total de l'extrait. Le traitement par voie orale pendant 28 jours consiste à un gavage cinq jours par semaine et une administration dans l'eau de boisson deux jours par semaine.

Les animaux de tous les groupes ont été testés pour leur capacité fonctionnelle in situ (les deux pattes postérieures) grâce à des mesures de la force maximale isométrique absolue et spécifique du muscle tibial antérieur (TA) (Figures 6A et 6B) après 7 jours d'immobilisation (n=6 souris, 2 valeurs par souris pour le groupe témoin (véhicule), n=6 souris, deux valeurs par souris pour le groupe test (BIO101), après 14 jours d'immobilisation suivie de deux semaines de remobilisation (n=6 par souris, deux valeurs par souris pour le véhicule (groupe témoin), n=6 souris, deux valeurs par souris pour le composé BIO101), et après 14 jours d'immobilisation suivie de deux semaines de remobilisation (n=6 par souris, deux valeurs par souris pour le véhicule (groupe témoin), n=6 souris, deux valeurs par souris pour le composé BIO101).

### Poids des muscles tibial antérieur (TA) et gastrocnémien (Figure 4)

Le poids des muscles TA (Figure 4A) et gastrocnémien (Figure 4B) ont été évalués chez des souris non immobilisées (groupe contrôle), et après 14 jours d'immobilisation chez des souris traitées soient par le véhicule soit par le composé BIO101 durant toute la durée de l'immobilisation. De manière attendue, on observe que l'immobilisation provoque une diminution de la masse musculaire du TA (-21.7%, p<0,001) chez des souris ayant reçu le véhicule par rapport au groupe contrôle, non immobilisé (Figure 4A).

On observe que le poids des muscles TA et gastrocnémien ne varie pas significativement dans le groupe test des souris traitées avec le composé BIO101 par rapport au groupe témoin traité véhicule (Figure 4A et 4B).

### Force maximale isométrique absolue et spécifique du muscle tibial antérieur (étude fonctionnelle in situ (Figures 5 et 6)

Une évaluation de la contractilité in situ du muscle TA est réalisée à différents temps du protocole : sur des souris contrôles non immobilisées (groupe contrôle, J0), sur des souris soumises à une immobilisation des pattes postérieure pendant 7 jours (J7), 14 jours (J14), immobilisées 14 jours puis remobilisées 2 semaines (J28).

Lorsque l'on considère la force développée par le muscle TA après sept jours d'immobilisation, de manière attendue, on constate que les animaux traités avec le véhicule (groupe témoin) ont une force de contraction maximale isométrique absolue significativement moins importante que celle des animaux contrôle non immobilisés (-34,7%, p<0,001) (Figure 5A). Les animaux traités avec le composé BIO101 présentent une perte de force absolue moins importante (-21,1%, p=0,001) par rapport au contrôle, que les animaux traités avec le véhicule (Figure 5A).

De manière intéressante, on observe que le traitement par le composé BIO101 permet aux animaux immobilisés pendant 7 jours de conserver une force maximale isométrique absolue significativement plus importante et améliore leur performance (+21%, p=0,01) par rapport à des animaux traités par le véhicule, et cela malgré l'absence d'effet du composé BIO101 sur la perte de masse.

On observe que les animaux traités avec le véhicule ont une force de contraction maximale isométrique spécifique (sP0 ; Figure 5B) significativement moins importante que celle des animaux contrôle non immobilisés (-13,2%, p<0,01). De manière remarquable, la force maximale isométrique spécifique des animaux traités avec le composé BIO101 n'est pas significativement affectée par 7 jours d'immobilisation : en effet, le traitement par le composé BIO101 permet aux animaux immobilisés pendant 7 jours de conserver une fonction musculaire normale par rapport aux animaux du groupe immobilisés, traités avec le véhicule (+24.3%, p<0,001).

Au moment de la levée de l'immobilisation, à J14, les souris ayant reçu le traitement BIO101 n'ont perdu que 22,4% (p<0.001) de la force isométrique maximale absolue par rapport à des souris contrôle non immobilisées (J0), contre 34% (p<0.001) pour les souris ayant reçu le véhicule. Le traitement par BIO101 permet de limiter la perte de force maximale isométrique absolue (+17,5%, p<0,05) par rapport à des souris traitées avec le véhicule (Figure 6A).

Concernant la force maximale isométrique spécifique, les souris ayant reçu le traitement BIO101 ne perdent pas de force 14 jours post immobilisation par rapport aux souris contrôle non immobilisées (+5%, 2,94g/mg versus 2,80g/mg respectivement, p=ns).

Les souris traitées par le véhicule, quant à elles, perdent 11,3% de leur force spécifique par rapport aux souris non immobilisées (p=0,06).

Après 14 jours d'immobilisation, le traitement par BIO101 tend à limiter la perte de force maximale spécifique (+18,4%, ns) par rapport à des souris traitées avec le véhicule (Figure 6B).

### Conclusion

Compte tenu des propriétés des phytoecdysones et leurs dérivés sur la fonction musculaire de mammifères soumis à une immobilisation, l'utilisation des phytoecdysones et leurs dérivés peut donc être proposée, pour préserver la fonction musculaire, notamment en ce qui concerne la force musculaire et ainsi ralentir la perte des fonctions musculaires liée à une immobilisation.

### Références

Alkner BA, Tesch PA. Knee extensor and plantar flexor muscle size and function following 90 days of bed rest with or without resistance exercise. Eur J Appl Physiol. 2004, 93(3):294-305
Appell HJ. Muscular atrophy following immobilisation. A review. Sports Med 1990, 10:42-58.
Bar-Shai M, Carmeli E, Coleman R, Rozen N, Perek S, Euchs E, Reznick AZ. The effect of hindlimb immobilization on acid phosphatase, metalloproteinase and nuclear factor-kappaB in muscles of young and old rats. Mech Ageing Dev. 2005, 126:289-97.
Berg HE, Eiken O, Miklavcic L, Mekjavic IB. Hip, thigh and calf muscle atrophy and bone loss after 5-week bedrest inactivity. Eur J Appl Physiol. 2007, 99(3):283-9.
Bigard AX, Boehm E, Veksler V, Mateo P, Anflous K, Ventura-Clapier R. Muscle unloading induces slow to fast transitions in myofibrillar but not mitochondrial properties. Relevance to skeletal muscle abnomalies in heart failure. J Physiol. 1998, 548:649-61.
Bodine SC. Disuse-induced muscle wasting. Int J Biochem Cell Biol. 2013, 45(10):2200-8.
De Jonghe B, Bastuji-Garin S, Durand MC, Malissin I, Rodrigues P, Cerf C, Outin H, Sharshar T. Respiratory weakness is associated with limb weakness and delayed weaning in critical illness. Groupe de Réflexion et d'Etude des Neuromyopathies en Réanimation. Crit Care Med. 2007, 35(9):2007-15.
Gillespie L.D., Robertson M.C., Gillespie W.J., Sherrington C., Gates S., et al. Interventions for preventing falls in older people living in the community. Cochrane Database Syst Rev 2012, 9: CD007146.
Gorelick-Feldman J, MacLean D, Ilic N, Poulev A, Lila MA, Cheng D, Raskin I. Phytoecdysteroids increase protein synthesis in skeletal muscle cells. J Agric Food Chem 2008, 56: 3532-3537.
Grosset JF, Onambele-Pearson G. Effect of foot and ankle immobilization on leg and thigh muscles' volume and morphology: a case study using magnetic resonance imaging. Anat Rec (Hoboken). 2008, 291(12):1673-83.
Hermans G, Van den Berghe G. Clinical review: intensive care unit acquired weakness. Crit Care. 2015, 19:274
Hunter RB, Stevenson E, Koncarevic A, Mitchell-Felton H, Essig DA, Kandarian SC. Activation of an alternative NF-kappaB pathway in skeletal muscle during disuse atrophy. FASEB J. 2002, 16:529-38
INSERM, Expertise collective : Activité physique et prévention des chutes chez les personnes âgées. Les éditions Inserm, 2015, Paris.
lordens GI, Van Lieshout EM, Schep NW, De Haan J, Tuinebreijer WE, Eygendaal D, Van Beeck E, Patka P, Verhofstad MH, Den Hartog D, FuncSiE Trial Investigators. Early mobilisation versus plaster immobilisation of simple elbow dislocations: results of the FuncSiE multicentre randomised clinical trial. Br J Sports Med. 2017, 51(6):531-538.
Klop C, Welsing PM, Harvey NC, Cooper C, Elders PJ, Bijlsma JW, Leufkens HG, de Vries F. Mortality in British hip fracture patients, 2000-2010: a population-based retrospective cohort study. Bone, 2014, 66:171-7.
Lang SM, Kazi AA, Hong-Brown L, Lang CH. Delayed Recovery of Skeletal Muscle Mass following hindlimb immobilization in mTOR Heterozygous Mice. Plos One, 2012, 7(6):e38910
Lawrence MM. 2012. Ajuga turkestanica as a countermeasure against sarcopenia and dynapenia. Ms thesis, Appalachian State University
Lebrasseur NK, Schelhorn TM, Bernardo BL, Cosgrove PG, Loria PM, Brown TA. Myostatin inhibition enhances the effects on performance and metabolic outcomes in aged mice. J Gerontol A Biol Sci Med Sci. 2009; 64:940-48.
Lexell J. Ageing and human muscle: observations from Sweden. Can J Appl Physiol. 1993; 18:2-18
Lund CA, Møller AM, Wetterslev J, Lundstrøm LH. Organizational factors and long-term mortality after hip fracture surgery. A cohort study of 6 143 consecutive patients undergoing hip fracture surgery. PLoS One, 2014, 9(6):e99308.
Lynch GS, Schertzer JD, Ryall JG. Therapeutic approaches for muscle wasting disorders. Pharmacology & Therapeutics 2007; 113:461-87.
Milsom J, Barreira P, Burgess DJ, Iqbal Z, Morton JP. Case study: Muscle atrophy and hypertrophy in a premier league soccer player during rehabilitation from ACL injury. Int J Sport Nutr Exerc Metab. 2014, 24(5):543-52.
Narici MV, Maffulli N. Sarcopenia: characteristics, mechanisms and functional significance. Br Med Bull. 2010; 95:139-59.
Nilwik R, Snijders T, Leenders M, Groen BB, van Kranenburg J, Verdijk LB, van Loon LJ. The decline in skeletal muscle mass with aging is mainly attributed to a reduction in type II muscle fiber size. Exp Gerontol. 2013, 48(5):492-8.
Ohira Y, Yoshinaga T, Ohara M, Kawano F, Xiao DW, Higo Y, Terada M, Matsuoka Y, Roy RR. The role of neural and mechanical influences in maintaining normal fast and slow muscle properties. Cells Tissues Organs 2006, 182: 129-42.
Onambele GL, Narici MV, Maganaris CN. Calf muscle-tendon properties and postural balance in old age. J Appl Physiol, 2006, 100:2048-2056.
Phillips T, Leeuwenburgh C. Muscle fiber-specific apoptosis and TNF-α signaling in sarcopenia are attenuated by life-long calorie restriction. FASEB J. 2005, 19:668-70.
Romanick M, Brown-Borg HM. Murine models of atrophy, cahchexia, and sarcopenia in skeletal muscle. Biochim Biophys Acta 2013, 1832(9):1410-20.
Sakuma K, Yamaguchi A. Sarcopenia and cachexia: the adaptations of negative regulators of skeletal muscle mass. J Cachexia Sarcopenia Muscle. 2012, 3:77-94.
Siriett V, Platt L, Salerno MS, Ling N, Kambadur R, Sharma M. Prolonged absence of myostatin reduces sarcopenia. J Cell Physiol. 2006, 209:866-73.
Suetta C, Magnusson SP, Rosted A, Aagaard P, Jakobsen AK, Larsen LH, Duus B, Kjaer M. Resistance training in the early postoperative phase reduces hospitalization and leads to muscle hypertrophy in elderly hip surgery patientsa controlled, randomized study. J Am Geriatr Soc. 2004, 52(12):2016-22.
Syrov VN. Comparative experimental investigations of the anabolic activity of ecdysteroids and steranabols. Pharm Chem Journal 2000, 34(4):193-7.
Tóth N, Szabó A, Kacsala P, Héger J, Zádor E. 20-Hydroxyecdysone increases fiber size in a muscle-specific fashion in rat. Phytomedicine. 2008,15(9):691-8.
Wentworth LJ, Bechtum EL, Hoffman JG, Kramer RR, Bartel DC, Slusser JP, Tilbury RT. Decreased bed rest post-percutaneous coronary intervention with a 7-French arterial sheath and its effects on vascular complications. J Clin Nurs. 2018, 27(1-2):e100-e115.
Yamada E, Bastie CC, Koga H, Wang Y, Cuervo AM, Pessin JE. Mouse skeletal muscle fiber-type-specific macroautophagy and muscle wasting are regulated by a Fyn/STAT3/ Vps34 signaling pathway. Cell Rep. 2012, 1 (5):557-69.

## Revendications

1. - Composition comportant au moins :
- la 20-hydroxyecdysone ; ou,
- au moins un composé de formule générale (I) : 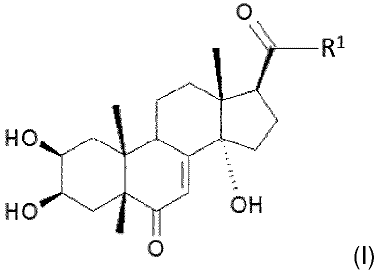 dans lequel :
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement choisi parmi OH, OMe, (C₁-C₆), N(C₁-C₆), CO₂(C₁-C₆) ; un groupement CH₂Br,
**W** étant un hétéroatome choisi parmi N, O et S, de préférence O et encore plus préférentiellement S ; ou,
- Au moins un composé de formule (II) : 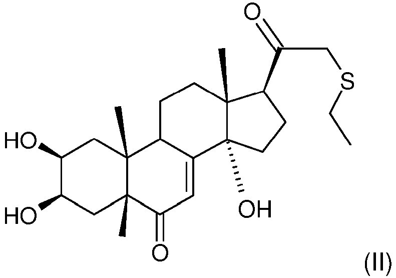 pour son utilisation chez le mammifère pour la prévention de la perte de force musculaire lors de l'immobilisation.

2. **-** Composition pour son utilisation selon la revendication 1, qui comporte un composé choisi parmi les composés suivants :
**n° 1 :** (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one,
**n° 2 :** (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
**n° 3 :** (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
**n° 4 :** (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
**n° 5 :** (2S,3R,5R,10R,13R,14S,17S)-17-[2-(3-diméthylaminopropyl (méthyl)amino)acétyl]-2,3,14-trihydroxy-10,13-diméthyl-2,3,4,5,9,11,12,15,16,17-décahydro-1H-cyclopenta[a]phénanthrèn-6-one;
**n° 6 :** 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
**n°** 7 : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
**n° 8 :** (2S,3R,5R,10R,13R,14S,17S)-2,3,14-trihydroxy-17-[2-(2-hydroxyéthyl sulfanyl)acétyl]-10,13-diméthyl-2,3,4,5,9,11,12,15,16,17-décahydro-1H cyclopenta[a]phénanthrèn-6-one.

3. **-** Composition pour son utilisation selon l'une des revendications précédentes, sous une forme incorporée à une formulation pharmaceutique acceptable convenant pour une administration par voie orale.

4. **-** Composition pour son utilisation selon l'une quelconque des revendications précédentes, dans laquelle les phytoecdysones sont administrées à une dose comprise entre 50 et 1000 milligrammes par jour chez l'humain.

5. **-** Composition pour son utilisation selon l'une quelconque des revendications précédentes, administrée lors de l'immobilisation.

6. **-** Composition pour son utilisation selon l'une quelconque des revendications précédentes, administrée jusqu'à la levée de l'immobilisation.

7. **-** Composition pour son utilisation selon l'une quelconque des revendications 5 ou 6, administrée en outre au cours d'une durée prédéterminée après la levée de l'immobilisation.

## Patentansprüche

1. - Zusammensetzung, die mindestens Folgendes umfasst:
- 20-Hydroxyecdyson; oder
- mindestens eine Verbindung der allgemeinen Formel (I): 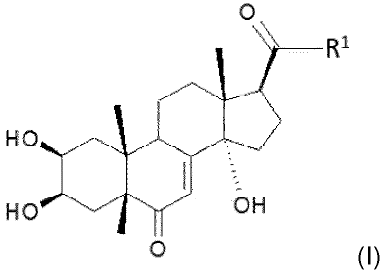 wobei:
**R¹** ausgewählt ist aus: einer Gruppe (C₁-C₆)**W**(C₁-C₆); einer Gruppe (C₁-C₆)**W**(C₁-C₆)**W**(C₁-C₆); einer Gruppe (C₁-C₆)**W**(C₁-C₆)CO₂(C₁-C₆); einer Gruppe (C₁-C₆)**A**, wobei **A** einen Heterocyclus darstellt, der gegebenenfalls durch eine Gruppe substituiert ist, die ausgewählt ist aus OH, OMe, (C₁-C₆), N(C₁-C₆), CO₂(C₁-C₆); einer Gruppe CH₂Br,
wobei **W ein** Heteroatom ist, das aus N, O und S, vorzugsweise O und noch bevorzugter S, ausgewählt ist; oder
- mindestens eine Verbindung der Formel (II): 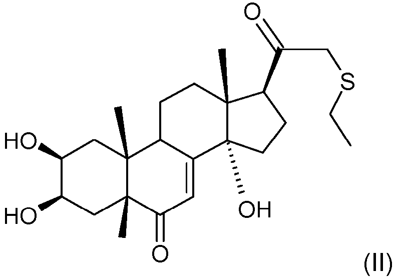 zur Verwendung bei Säugetieren zur Verhinderung von Muskelkraftverlust während der Immobilisierung.

2. **-** Zusammensetzung zur Verwendung nach Anspruch 1, die eine Verbindung umfasst, die aus den folgenden Verbindungen ausgewählt ist:
Nr. 1: (2S,3R,5R,10R,13R,14S,17S)-2,3,14-Trihydroxy-10,13-dimethyl-17-(2-morpholinoacetyl)-2,3,4,5,9,11,12,15,16,17-decahydro-1H-cyclopenta[a]phenanthren-6-on,
Nr. 2: (2S,3R,5R,10R,13R,14S,17S)-2,3,14-Trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-on;
Nr. 3: (2S,3R,5R,10R,13R,14S,17S)-2,3,14-Trihydroxy-17-[2-(4-hydroxy-1-piperidyl)acetyl]-10,13-dimethyl-2,3,4,5,9,11,12,15,16,17-decahydro-1H-cyclopenta[a]phenanthren-6-on;
Nr. 4: (2S,3R,5R,10R,13R,14S,17S)-2,3,14-Trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-on;
**Nr. 5:** (2S,3R,5R,10R,13R,14S,17S)-17-[2-(3-Dimethylaminopropyl (methyl)amino)acetyl]-2,3,14-trihydroxy-10,13-dimethyl-2,3,4,5,9,11,12,15,16,17-decahydro-1H-cyclopenta[a]phenanthren-6-on;
**Nr. 6:** 2-[2-Oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]sulfanylethylacetat;
**Nr. 7:** (2S,3R,5R,10R,13R,14S,17S)-17-(2-Ethylsulfanylacetyl)-2,3,14-trihydroxy-10,13-dimethyl-2,3,4,5,9,11,12,15,16,17-decahydro-1H-cyclopenta[a]phenanthren-6-on;
**Nr. 8:** (2S,3R,5R,10R,13R,14S,17S)-2,3,14-Trihydroxy-17-[2-(2-hydroxyethyl sulfanyl)acetyl]-10,13-dimethyl-2,3,4,5,9,11,12,15,16,17-decahydro-1H cyclopenta[a]phenanthren-6-on.

3. **-** Zusammensetzung zur Verwendung nach einem der vorhergehenden Ansprüche in einer Form, die in eine akzeptable pharmazeutische Formulierung integriert ist, die für die orale Verabreichung geeignet ist.

4. **-** Zusammensetzung zur Verwendung nach einem der vorhergehenden Ansprüche, wobei die Phytoecdysonen in einer Dosis zwischen 50 und 1000 Milligramm pro Tag beim Menschen verabreicht werden.

5. **-** Zusammensetzung zur Verwendung nach einem der vorhergehenden Ansprüche, die während der Immobilisierung verabreicht wird.

6. **-** Zusammensetzung zur Verwendung nach einem der vorhergehenden Ansprüche, die bis zur Aufhebung der Immobilisierung verabreicht wird.

7. **-** Zusammensetzung zur Verwendung nach einem der Ansprüche 5 oder 6, die ferner über eine vorbestimmte Zeit nach dem Aufheben der Immobilisierung verabreicht wird.

## Claims

1. - Composition including at least:
- 20-hydroxyecdysone; or,
- at least one compound of general formula (I): 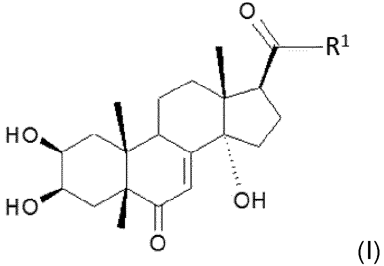 wherein:
**R¹** is chosen from: a (C₁-C₆)**W**(C₁-C₆) group; a (C₁-C₆)**W**(C₁-C₆)**W**(C₁-C₆) group; a (C₁-C₆)**W**(C₁-C₆)CO₂(C₁-C₆) group; a (C₁-C₆)**A** group, A representing a heterocycle optionally substituted by a group chosen from **OH,** OMe, (C₁-C₆), N(C₁-C₆), CO₂(C₁-C₆); a CH₂Br group;
**W** being a heteroatom chosen from N, O and S, preferably O and even more preferentially S; or,
- at least a compound of formula (II): 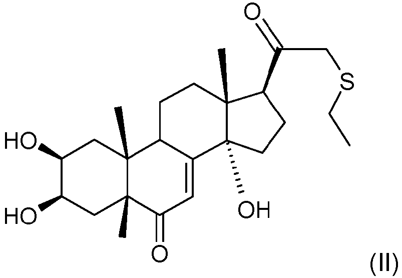 for use thereof in mammals for preventing loss of muscle strength during immobilisation.

2. **-** Composition for use thereof according claim 1, which comprises a compound chosen from the following compounds:
**n° 1:** (2S,3R,5R,10R,13R,14S,17S)-2,3,14-trihydroxy-10,13-dimethyl-17-(2-morpholinoacetyl)-2,3,4,5,9,11,12,15,16,17-decahydro-1H-cyclopenta[a]phenanthren-6-one,
**n° 2:** (2S,3R,5R,10R,13R,14S,17S)-2,3,14-trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-one;
**n° 3:** (2S,3R,5R,10R,13R,14S,17S)-2,3,14-trihydroxy-17-[2-(4-hydroxy-1-piperidyl)acetyl]-10,13-dimethyl-2,3,4,5,9,11,12,15,16,17-decahydro-1H-cyclopenta[a]phenanthren-6-one;
**n° 4:** (2S,3R,5R,10R,13R,14S,17S)-2,3,14-trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-one;
**n° 5:** (2S,3R,5R,10R,13R,14S,17S)-17-[2-(3-dimethylaminopropyl (methyl)amino)acetyl]-2,3,14-trihydroxy-10,13-dimethyl-2,3,4,5,9,11,12,15,16,17-decahydro-1H-cyclopenta[a]phenanthren-6-one;
**n° 6:** ethyl 2-[2-oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]sulfanylacetate;
**n° 7:** (2S,3R,5R,10R,13R,14S,17S)-17-(2-ethylsulfanylacetyl)-2,3,14-trihydroxy-10,13-dimethyl-2,3,4,5,9,11,12,15,16,17-decahydro-1H-cyclopenta[a]phenanthren-6-one;
**n° 8:** (2S,3R,5R,10R,13R,14S,17S)-2,3,14-trihydroxy-17-[2-(2-hydroxyethyl sulfanyl)acetyl]-10,13-dimethyl-2,3,4,5,9,11,12,15,16,17-decahydro-1H cyclopenta[a]phenanthren-6-one.

3. **-** Composition for use thereof according to any of the preceding claims, in a form incorporated in a pharmaceutically acceptable formulation suitable for oral administration.

4. **-** Composition for use thereof according to any one of the preceding claims, wherein the phytoecdysones are administered at a dose of between 50 and 1000 milligrams per day in humans.

5. - Composition for use thereof according to any one of the preceding claims, administered during immobilisation.

6. **-** Composition for use thereof according to any one of the preceding claims, administered until immobilisation ends.

7. **-** Composition for use thereof according to either one of claims 5 or 6, also administered during a predetermined period after ending of immobilisation.
